# EUROPEAN PATENT APPLICATION

(11) **EP 1 818 552 A1**
(43) Date of publication of application: **15.08.2007**
(21) Application number: 05793123.0
(22) Date of filing: 11.10.2005
(51) Int. Cl.: F16C 19/18, F16C 33/32, F16C 33/62

(54) **ULTRATHIN WALL ROLLING BEARING**

(30) Priority: 05.11.2004 JP 2004322419
(71) Applicant: NTN CORPORATION, Osaka-shi, Osaka 550-0003 (JP)
(72) Inventor: HIRAMATSU, KENGO, NTN CORPORATION, Kuwana-shi Mie, 511-0811 (JP); OYA, Yosuke, NTN CORPORATION, Kuwana-shi Mie, 511-0811 (JP)
(74) Representative: White, Duncan Rohan
(86) International application number: PCT/JP2005/018706
(87) International publication number: WO 2006/048996

(57) **Abstract**

An ultrathin-walled rolling bearing enabling prevention of fretting during transportation, an increase in moment rigidity of the bearing, and suppression of an effect of a centrifugal force generated in high-speed rotation. A material of the races (an outer member (10) and an inner member (20)) of the ultrathin-walled rolling bearing is allowed to be different from a material of rolling elements (balls (30) ) . For example, the races (10, 20) are formed of steel, and the rolling elements (30) are formed of a material having a modulus of direct elasticity of 1. 5 times that of the steel or higher. Alternatively, the races (10, 20) are formed of steel, and the rolling elements (30) are formed of a material having a density of 1/2 or less of that of the steel.

## Description

### Technical Field

The present invention relates to an ultrathin-walled rolling bearing for use in an industrial robot, a machine tool, medical equipment or the like.

### Background Art

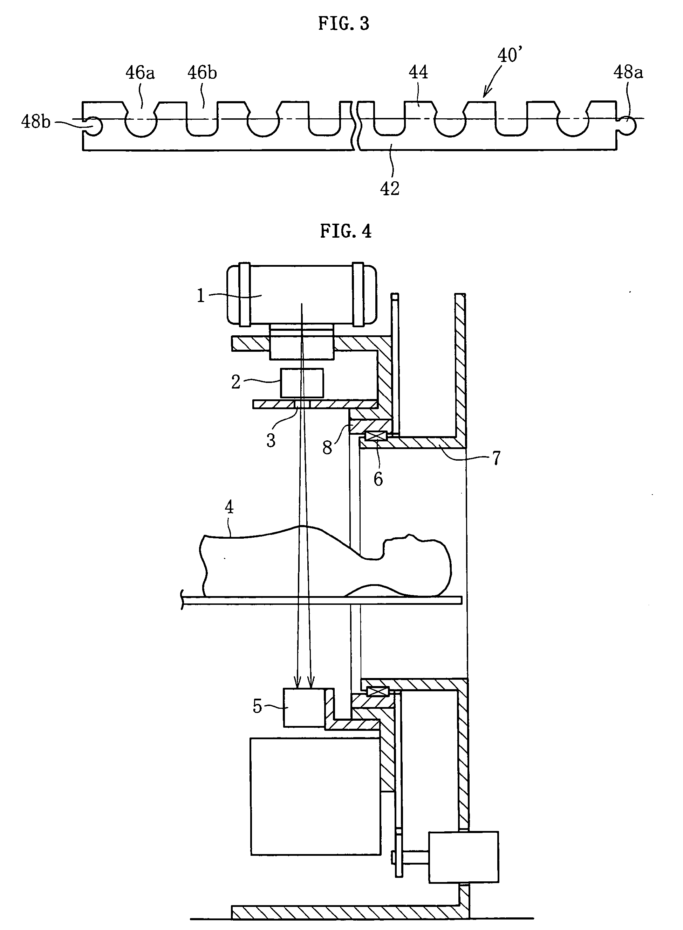

FIG. 4 shows a CT scanner device, an example of one kind of medical equipment. In the CT scanner device, an X ray generated by an X ray tube assembly 1 is irradiated onto an object 4 through a wedge filter 2 for uniformizing intensity distribution thereof and a slit 3 for restricting the intensity distribution. The X ray passing through the object 4 is received by a detector 5, converted into an electrical signal and transferred to a computer (not shown). Components such as the X ray tube assembly 1, the wedge filter 2, the slit 3, and the detector 5 are mounted on a substantially cylindrical rotary pedestal 8 supported rotatably around a stationary pedestal 7 through a bearing 6, and rotate around the object 4 through rotation of the rotary pedestal 8. In this way, by rotating the X ray tube assembly 1 and the detector 5 opposing to each other around the object 4, projection data covering all angles at every point within a cross section of the object 4 to be examined is obtained, and a cross sectional image is obtained based on these pieces of data using a pre-programmed reconstruction program.

In the CT scanner device, an inner circumferential surface of the stationary pedestal 7 has a large opening of approximately 1 m in diameter to let the object 4 enter therein, and therefore, a so-called ultrathin-walled rolling bearing having a cross section which is significantly small relative to the diameter is used as the bearing 6 between the stationary pedestal 7 and the rotary pedestal 8.
Patent Document 1: JP-A-2000-329143
Patent Document 2: JP-A-2001-304266
Patent Document 3: JP-A-2002-081442

### Disclosure of the Invention

### Problems to be solved by the Invention

A CT scanner device is a device, which applies an X ray to a human subject while rotating therearound and detects the X ray passing through the human subject, for obtaining a cross-sectional tomogram view by processing projection information by a computer. To enable shortening of exposure time and photographing of an organ which moves fast such as a heart, there is a market need for a bearing used for the CT scanner, capable of rotating at higher speed.

Further, it is inevitable for quality models to allow a weight increase of a rotating portion for higher functions such as obtainment of images of high definition and shooting of dynamic images. For endurance against the weight increase, it is required to increase a moment rigidity.

Further, fretting caused during transportation of the bearing, especially during transportation to overseas or the like may result in reduction in life thereof due to abrasion triggered by the fretting.

It is an object of the present invention to solve the above-mentioned problems involved in the ultrathin-walled rolling bearing.

### Means for solving the Problems

An ultrathin-walled rolling bearing according to the present invention, includes: an outer member provided with, in an inner circumferential surface thereof, two rows of raceways on which rollingelements roll; an inner member which is positioned on an inner circumferential side of the outer member and includes an annular member and an engaging member engaging with an outer circumference of the annular member, an axial clearance t being interposed therebetween, the inner member being provided with raceways, which are formed in outer circumferential surfaces of the annular member and the engaging member, respectively, and on which the rollingelements roll; the rolling elements rollably interposed between the raceways of the outer member and the raceways of the inner member and having a diameter dw of which the ratio (dw/PCD) to a pitch circle diameter is 0.03 or less; and a preloading means for applying a preload by reducing an axial clearance t by pressing the engaging member, in which a material of the outer member and the inner member and a material of the rolling elements are different from each other.

The outer member and the inner member may be formed of steel and the rolling elements may be formed of a material having a modulus of direct elasticity of 1.5 times that of the steel or higher.

The outer member and the inner member may be formed of steel and the rolling elements may be formed of a material having a density of 1/2 or less of that of the steel.

Examples of the material satisfying one or both of a condition in which the modulus of direct elasticity is 1.5 times that of the steel or higher and a condition in which the density is 1/2 or less of that of the steel include ceramics and an acrylic resin.

### Effects of the Invention

According to the present invention, by adopting, as the material of the rolling elements, the material different from that of the races (outer member and inner member), it is possible to prevent the fretting during transportation.

Further, by adopting, as the material of the rolling elements, the material having the modulus of elasticity (Young's modulus) of 1.5 times that of the steel or higher, it is possible to increase a moment rigidity of the bearing by increasing the rigidity of the rolling elements. Accordingly, when a displacement due to a moment load application is the same, a larger moment load can be applied as compared to the conventional art.

Further, by adopting, as the material of the rolling elements, a material having the density of 1/2 or less of that of the steel, the weight of the rolling elements is reduced, thereby making it possible to suppress an effect of a centrifugal force caused upon high-speed rotation, that is, generation of overstress.

### Brief Description of the Drawings

FIG. 1 is a sectional view of an ultrathin-walled rolling bearing according to an embodiment of the present invention.
FIG. 2 is an enlarged view of a main portion of FIG. 1.
FIG. 3 is an exploded plan view of a segment constituting a cage.
FIG. 4 is a sectional view of a CT scanner device.

### Description of Reference Numerals

- 10: outer member
- 12: raceway
- 14: female thread hole
- 16, 18: seal
- 20: inner member
- 22: annular member
- 24: small-diameter stage portion
- 26: raceway
- 28: engaging member
- 30: rolling element (ball)
- 40: cage
- 40': segment
- 42: base
- 44: columnar portion
- 46a, 46b: pocket
- 48a, 48b: connecting portion
- 50: presser member
- 52:
- S: preloading means

### Best Mode for carrying out the Invention

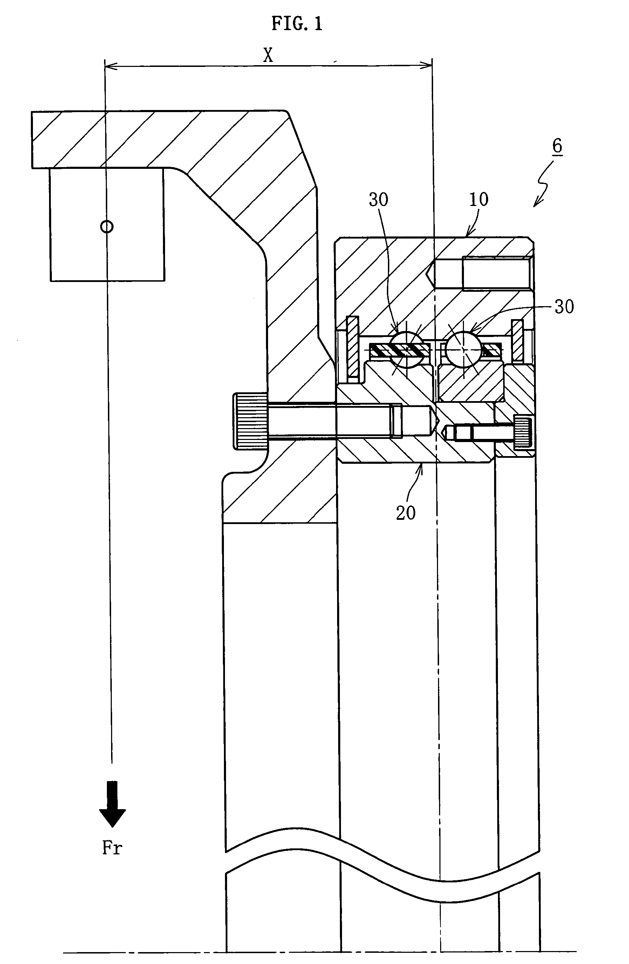

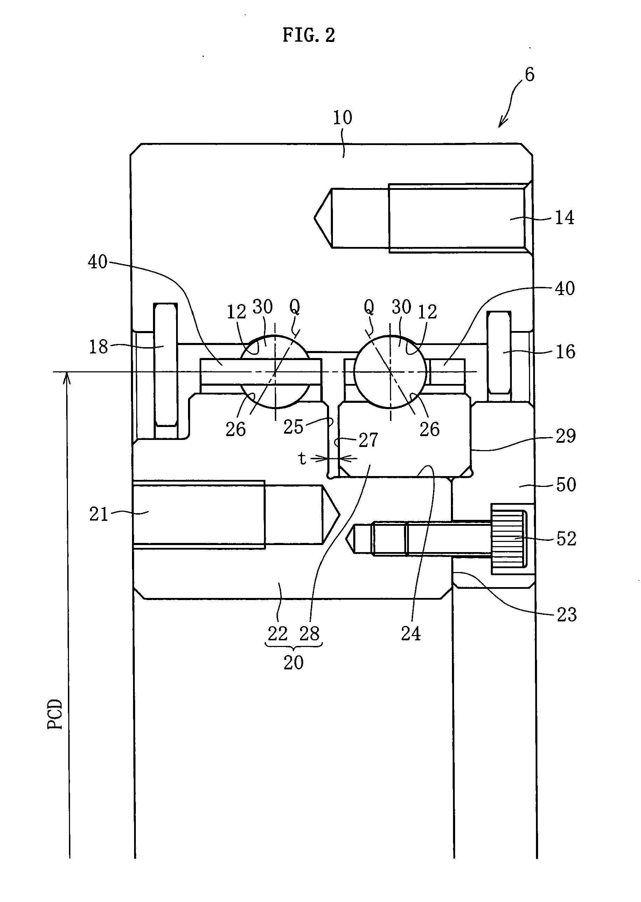

Hereinafter, a description will be made of an embodiment of the present invention with reference to the drawings. Here, FIG. 1 shows a structure of a bearing 6 of a CT scanner device shown in FIG. 4. FIG. 2 is an enlarged view of a main portion of FIG. 1.

As shown in FIG. 2, the bearing 6 includes, as main components, a ring-shaped outer member 10, a similar, ring-shaped inner member 20 concentrically disposed on an inner circumferential side of the outer member 10, two rows of rolling elements 30 interposed between the inner member 20 and the outer member 10, a cage 40 retaining the rolling elements 30 in the respective rows at equal intervals in a circumferential direction, seals 16 and 18 for sealing the openings at both ends of the bearing, and a preloading means S for applying a preload to the bearing.

The bearing 6 is a double row bearing having two rows of the rolling elements 30. Here, balls 30 as the rolling elements are arranged in two rows. There is illustrated a so-called double row angular contact ball bearing of a back-to-back arrangement, in which intersections of lines of action Q of a rolling element load is outside a pitch circle (a pitch circle diameter is represented by PCD). A contact angle, that is, an angle formed by a direction of the rolling element load and a plane perpendicular to a central axis of the bearing is set, for example, to 30°.

This double row angular contact ball bearing is an ultrathin-walled rolling bearing in which the ratio 0 = dB/PCD of the diameter dB of the ball 30 to the pitch circle diameter PCD is 0.03 or less (∅ ≤ 0.03). The diameter dB of the ball is set, for example, to 1/2 inch (12.7 mm), PCD is set to 1041.4 mm, and the ratio 0 is set to 0.012.

The outer member 10 includes two rows of raceways 12, on which the two rows of balls 30 rolls, formed in an inner circumferential surface thereof. The inner member 20 includes an annular member 22 and an engaging member 28. The annular member 22 has, at one end thereof, a small-diameter stage portion 24, and the engaging member 28 is engaged with the small-diameter stage portion 24. In each of outer circumferential surfaces of the annular portion 22 and the engaging portion 28, there is formed a raceway 26. The balls 30 are interposed between the two rows of raceways 12 of the outer member 10 and the two raceways 26 of the inner member 20, respectively.

The cage 40 is a separate type resin cage which is obtained by allowing a plurality of circular-shaped segments 40' to be connected in an annular shape. As shown in FIG. 3, the segment 40' includes a circular-shaped base 42 formed by dividing an annular body at a plurality of positions in a circumferential direction, a columnar portion 44 extending from the base 42 in a cantilever fashion, and a plurality of pockets 46a and 46b provided between the adjacent columnar portions 44. The columnar portion 44 extends in an axial direction beyond the pitch circle of the ball 30, which is indicated by the alternate long and short dash line in FIG. 3. The pockets 46a and 46b shown in the figure have two kinds of shapes. The two kinds of shapes include a shape of the first pocket 46a having its wall surface near an opening side (upper side of FIG. 3) of the pocket rather than a center of the pocket (positioned on the pitch circle in FIG. 3) shaped as a recessed circular surface when two-dimensionally viewed, and a shape of the second pocket 46b having an axially straight wall surface. The first pocket 46a and the second pocket 46b are provided alternately in the circumferential direction. Any of the wall surfaces of the pockets is a concave surface whose center of curvature is at the center of the pocket in a radial cross section (section perpendicular to a plane of FIG. 3).

The balls 3 are stored into the pockets 46a and 46b by pressing the balls 30 into the backside of the pockets from the openings of the pockets 46a and 46b. At this time, the balls 30 must be pressed in while forcing columnar portions 44 open on the inlet side, while no such operation is necessary for the second pocket 46b, so the process of incorporating the balls 30 into the cage 40 can be simplified. Note that the shapes and structures of the above-mentionedpockets 46a and 46b are only by way of illustration, and the pockets may be of the same shape, for example, in other words, pockets of various shapes and structures may be used depending upon the conditions or the like for using the bearing.

Both ends of each segment 40' are provided with connecting portions for connecting the adjacent segments to each other. Here, connecting portions 48a and 48b are shown for matingly engaging with corresponding connecting portions of the segments. The one connecting portion 48a has a raised shape broader on its tip side, and includes, as shown in the figure, for example, a substantially cylindrical portion extending in a radius direction of the cage and a neck portion having a narrower width than the cylindrical portion. The other connecting portion 48b has a recessed cylindrical shape which fits with the connecting portion 48a of the raised shape. When connecting the adjacent segments 40' to each other, the connecting portion (for example, 48a) of the one segment is pressed into the connecting portion (for example, 48b) of the other segment in the radius direction. As a result, the connecting portions 48a and 48b fit with each other, thereby preventing the segments 40' from being separated from each other in the circumferential direction.

The preloading means S is used to appropriately preload an inside of the bearing by pressurizing the engaging member 28 toward the inside of the bearing, and has the following construction, for example.

The annular member 22 and the engaging member 28 are engaged with each other in such a loose fit degree that those are moved by tightening by a presser member 50 described later. Before the presser member 50 is tightened, there is an axial clearance t between a shoulder portion 25 of the annular member 22 and an end surface 27 of the engaging member 28, facing the shoulder portion 25. The other end surface 29 of the engaging member 28 slightly axially projects from the end surface 23 of the annular member 22. A projection amount thereof is equal to or slightly larger than a width of the axial clearance t.

The outer member 10 has a female thread hole 14 formed in an end surface on a right side of FIG. 1. By screwing a bolt or the like (not shown) into the female thread hole 14, the outer member 10 is fixed to a rotary pedestal 8 of the CT scanner device shown in FIG. 3. Similarly, the inner member 20 has a female thread hole 21 formed in an end surface of the inner member 20 (of the annular member 22 in this embodiment) on a side opposite to the end surface of the outer member 10, in which the female thread hole 14 is provided. By screwing a bolt or the like (not shown) into the female thread hole 21, the inner member 20 is fixed to a stationary pedestal 7. Thus, the outer member 10 serves as a rotating side rotating together with the rotary pedestal 8, and the inner member 20 (annular member 22 and engaging member 28) serves as a non-rotating stationary side. Depending on a structure of the CT scanner device, conversely to the above description, the outer member 10 may be the non-rotating stationary side and the inner member 20 may be the rotating side rotating together with the rotary pedestal 8.

The ring-shaped presser member 50 is fixed, by using a fastening means 52 such as a bolt, to the end surface of the annular member 22 on the side opposite to the end surface provided with the female thread hole 21. An outer radial end of the presser member 50 is substantially in the same level as the outer circumferential surface of the engaging member 28. Of the pair of seals described above, one seal 16 is located at a position opposing the outer radial end of the presser member 50.

With the above-mentioned construction, when the fastening means 52 is fastened, the presser member 50 pressurizes and forces the engaging member 28 into the inside of the bearing. This allows the above-mentioned preloading means S to function and cause the axial clearance t to be reduced, and the pressurizing force is transmitted to the outer member 10 through the balls 3 so that the outer member 10 is pressed thereinto. As a result, a bearing clearance of both bearing portions is removed and the preload is applied. The width of the initial axial clearance t is predetermined so that a prescribed amount of preload is obtained at the point when the engaging member 28 is pressed in and the axial clearance t is 0, and thus the preload can be adjusted at high precision and by a simple operation. Alternatively, a method of controlling the tightening torque of the fastening means 52, for example, may be employed to apply a prescribed amount of preload to the bearing. In this case, the axial clearance t is not necessarily 0 after the application of a preload.

Different materials are adopted for races (outer member 10 and inner member 20) and the balls 30. Specifically, in this embodiment, the races are formed of steel. On the other hand, the balls 30 are composed of a material having a modulus of direct elasticity of 1.5 times that of steel or higher or a material having a density 1/2 or less of that of the steel. Examples of the material having such properties include ceramics (Si₃N₄) and an acrylic resin (PMMA). Table 1 shows a comparison of physical properties of the materials.

**[Table 1]**

| Items | Steel (SUJ) | Ceramics (Si3N4) | Acrylic resin (PMMA) |
|---|---|---|---|
| Density (gr/cm²) | 7.8 | 3.3 | 1.2 |
| Modulus of direct elasticity (kgf/cm²) | 21200 | 32000 | 33000 |

FIG. 1 shows an example of a schematic structure of a bearing for a CT scanner and a weight member of a rotating portion. Here, when a weight of the rotating portion is represented by Fr and a distance from a center of the bearing to the weight member is represented by X, a moment load of FrX acts on the bearing 6. By adopting the material having a modulus of elasticity (Young's modulus) of 1.5 times that of the steel or higher as the material of the balls 30, it is possible to increase a moment rigidity of the bearing by increasing the rigidity of the balls 30. Further, by adopting the material having the density 1/2 or less of that of the steel as the material of the balls 30, it is possible to reduce the weight of the balls 30 and suppress an effect of a centrifugal force caused upon high-speed rotation, that is, generation of overstress. Further, by adopting a material different from that of the races (10, 20) as the material of the balls 30, it is possible to prevent fretting during transportation.

## Claims

1. An ultrathin-walled rolling bearing, comprising:
an outer member provided with, in an inner circumferential surface thereof, two rows of raceways on which rolling elements roll;
an inner member which is positioned on an inner circumferential side of the outer member and includes an annular member and an engaging member engaging with an outer circumference of the annular member, an axial clearance being interposed therebetween, the inner member being provided with raceways, which are formed in outer circumferential surfaces of the annular member and the engaging member, respectively, and on which the rollingelements roll;
the rolling elements rollably interposed between the raceways of the outer member and the raceways of the inner member and having a diameter dw of which the ratio (dw/PCD) to a pitch circle diameter is 0.03 or less; and
a preloading means for applying a preload by reducing the axial clearance by pressing the engaging member,
wherein a material of the outer member and the inner member and a material of the rolling elements are different from each other.

2. An ultrathin-walled rolling bearing according to claim 1, wherein the outer member and the inner member are formed of steel and the rolling elements are formed of a material having a modulus of direct elasticity of 1.5 times that of the steel or higher.

3. An ultrathin-walled rolling bearing according to claim 1, wherein the outer member and the inner member are formed of steel and the rolling elements are formed of a material having a density of 1/2 or less of that of the steel.

4. An ultrathin-walled rolling bearing according to claim 1, wherein the material of the rolling elements is ceramics.

5. An ultrathin-walled rolling bearing according to claim 1, wherein the material of the rolling elements is an acrylic resin.
